# EUROPÄISCHE PATENTANMELDUNG

(11) **EP 0 679 409 A2**
(43) Veröffentlichungstag der Anmeldung: **02.11.1995**
(21) Anmeldenummer: 95110865.3
(22) Anmeldetag: 10.03.1988
(51) Int. Cl.: A61N 1/04, A61N 1/32, A61N 1/40

(54) **Elektrodenanordnung**

(30) Priorität: 26.03.1987 CH 1163/87
(62) Teilanmeldung aus: 88902114.3
(71) Anmelder: DYNAMIS Medizintechnik AG, CH-4143 Dornach (CH)
(72) Erfinder: Müller, Felix, CH-8803 Rüschlikon (CH)
(74) Vertreter: Troesch Scheidegger Werner AG

(57) **Zusammenfassung**

Eine Elektrodenanordnung (41) für die Uebertragung oder Ableitung von elektrischen Signalen auf oder von einem Körper eines Lebewesens umfasst eine thermisch mit einem Oberflächenbereich der Anordnung gekoppeltes Heiz- und/oder Kühlanordnung (44a).

## Beschreibung

Die vorliegende Erfindung betrifft Elektrodenanordnungen für die Uebertragung oder Ableitung von elektrischen Signalen auf oder von einem Körper eines Lebewesens.

Für die Elektrobehandlung von Menschen oder Tieren ist es bekannt, letzteren, über Elektrodenanordnungen, elektrische Reizsignale aufzuschalten. Dabei werden üblicherweise kontinuierliche Impulszüge aufgeschaltet, sei dies in Form von Rechteckimpulsen, Dreieckimpulsen, Trapezimpulsen oder von Sinus-Signalen.

Es sind Elektrodenanordnungen für die Uebertragung oder Ableitung von elektrischen Signalen auf oder von einem Körper eines Lebewesens bekannt, insbesondere für die Uebertragung von Reizsignalen auf Körper, die relativ grossflächig leitende Kontaktoberflächen aufweisen.

Eine mittels elektrischer Reizsignale zu behandelnde Körperpartie bedarf jeweils einer gewissen "Anwärm"-Zeit, bis sie - ähnlich dem Aufwärmen vor sportlichen Leistungen - risikolos mit voller beabsichtigter Intensität behandelt werden kann.

Somit ist es eine Aufgabe der vorliegenden Erfindung, eine Elektrodenanordnung zu schaffen, welche gut definierte, reproduzierbare Impedanzverhältnisse bei raschem Auflegen auf eine Körperpartie sicherstellt und die Behandlungszeit zu verkürzen bzw. den Behandlungseffekt zu erhöhen.

Dies wird dadurch erreicht, dass die Elektrodenanordnung, welche einen flächigen, forminstabilen, elektrisch isolierenden Träger und mindestens einen flächigen, forminstabilen, elektrisch leitenden Kontaktbereich an der Trägeroberfläche sowie einen elektrischen Anschluss für den Kontaktbereich umfasst, erfindungsgemäss eine thermisch mit einem Oberflächenbereich der Anordnung gekoppelte Heiz- und/oder Kühlanordnung umfasst. Damit wird eine Verkürzung der Behandlungszeit bzw. des Aufwandes, bis eine Behandlung begonnen werden kann, erreicht.

Vorsehen einer Kühl- wie auch einer Heizanordnung kann dabei gewisse Therapiearten ermöglichen, während Vorsehen der Heizanordnung weiter, nach dem oben Gesagten, ermöglicht, die Behandlungszeit zu verkürzen.

Weitere bevorzugte Ausführungsformen der Elektrodenanordnungen sind in den Ansprüchen 3 bis 8 spezifiziert.

Die Erfindung wird nun beispielsweise anhand von Figuren erläutert.
Es zeigen:
- Fig. 1, 1a: je eine Elektrodenanordnung,
- Fig. 2: einen Ausschnitt aus der Elektrodenanordnung gemäss Fig. 1 in erfindungsgemässer Ausführung,
- Fig. 3 und 4: eine weitere erfindungsgemässe Elektrodenanordnung mit Elektrodenheizung.

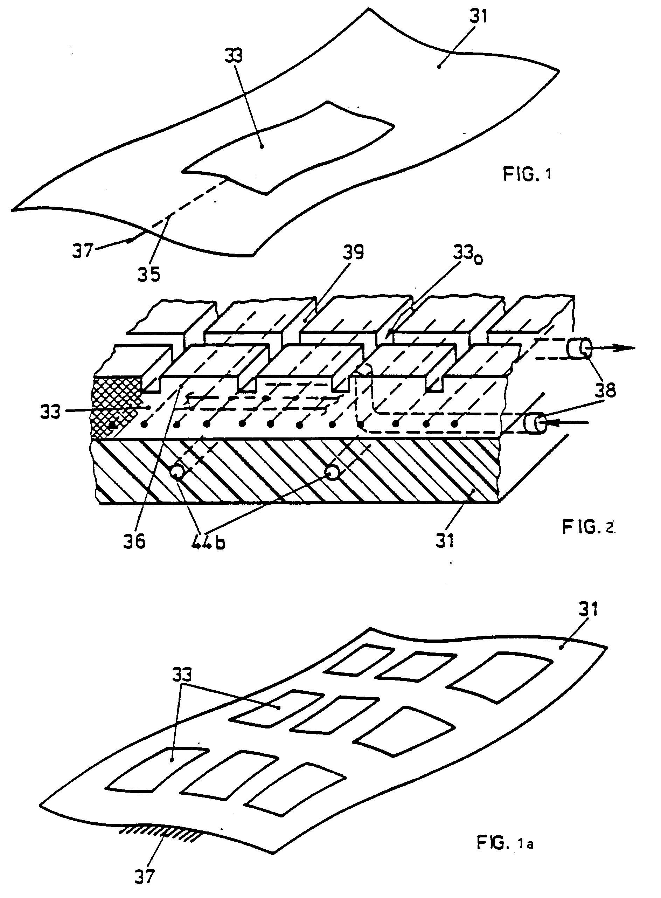

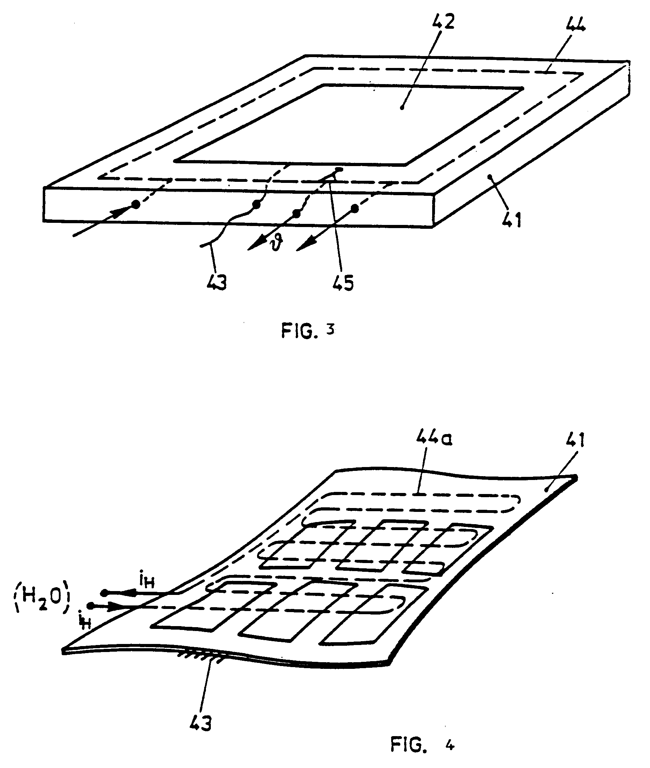

In Fig. 1 ist, in perspektivischer Darstellung eine Elektrodenanordnung dargestellt. Fig. 2 zeigt einen Teilausschnitt aus einer bevorzugten Ausführungsform der Anordnung gemäss Fig. 1. Sie umfasst einen flächigen, gummielastisch flexiblen Träger 31 aus elektrisch isolierendem Material. Dabei kann es sich vorzugsweise um Silikongummi handeln. In diesen Träger 31 sind ein, gemäss Fig. 1a mehrere elektrisch leitende Kontaktbereiche 33 so eingebettet, dass sie an der einen Oberfläche des Trägers 31 freiliegen. Auch der bzw. die Kontaktbereiche 33 sind forminstabil, vorzugsweise aus gummielastischem Material, wie aus leitendem Kunststoff gefertigt. Pro Bereich 33 ist ein elektrischer Anschluss 35, vorzugsweise innerhalb des Trägers 31 nach aussen geführt, zu einem Anschlussstecker 37. An ein und demselben gummielastischen Träger 31 können mehrere oder eine Vielzahl von Kontaktbereichen 33 vorgesehen sein mit entsprechender Anzahl elektrischer Anschlüsse 35, 37.

Wie aus Fig. 2 ersichtlich, wird bevorzugterweise die elektrische Kontaktierung der bevorzugterweise aus leitendem Kunststoff gefertigten Bereiche 33 mittels eines forminstabilen, flexiblen Leitergeflechtes 36 wie aus hochflexibler Hochfrequenzlitze realisiert. Dieses Geflecht 36 wird dabei entweder im leitenden Kunststoff eingebettet, wie in Fig. 2 dargestellt, oder in die Zwischenfläche zwischen Träger 31 und Bereich 33 eingelegt.

Wie in Fig. 2 dargestellt, ist bevorzugterweise der Bereich 33 mit Einnehmungen, vorzugsweise mit einem Rinnenmuster 39, versehen. Es sind dabei erfindungsgemäss flexible Röhrchen 38 durch den Träger 31 an den Oberflächenbereich 33ₒ der Bereiche 33 geführt, wodurch beispielsweise zur Konstanthaltung des Hautübergangswiderstandes bei zeitvariantem Schwitzen mittels einer zugeführten Elektrolytlösung oder zur Kühlung des Hautkontaktbereiches ein Flüssigmedium durchgetrieben werden kann.

Eine solche Elektrodenanordnung kann mit geringstem Aufwand und reproduzierbarem, optimal kleinem Uebergangswiderstand auf einen zu behandelnden Körperteil gelegt werden und schmiegt sich dort mit dem bzw. den Kontaktbereichen 33 flexibel an. Eine solche Elektrodenanordnung kann nicht nur im Rahmen der oben beschriebenen Elektrobehandlung mittels elektrischer Reizsignale eingesetzt werden, sondern auch als Abnehmerelektrode für die Messung gewisser biologischer, elektrischer Grössen, wie von Hautwiderständen.

In Fig. 3 ist eine weitere erfindungsgemässe Elektrodenanordnung dargestellt. Sie umfasst prinzipiell einen Träger 41, der formstabil oder, bevorzugterweise wie der Täger 31 von Fig. 1 oder 2, gummielastisch flexibel sein kann. Im weiteren sind, wiederum formstabil oder, bevorzugterweise gummielastisch forminstabil, ein oder mehrere Kontaktierungsbereiche 42 in Analogie zum Bereich 33 in den Fig. 1 und 2 vorgesehen, mit elektrischen Anschlüssen 43. Im Träger 41 ist nun eine grossflächig wirkende Heiz- oder Kühlanordnung 44 vorgesehen. Gemäss Fig. 4 handelt es sich um eine mäanerförmig im Träger 41 eingelassene Heizwendel 44a, welche extern mit einem Heizstrom i_{H} betrieben wird, oder um eine mäanderförmig im Träger 41 eingelassene flexible Leitungsanordnung 44b, wie in Fig. 2 dargestellt, für ein flüssiges Heiz- oder Kühlmedium, wie Wasser. Mittels eines oder mehrerer Temperaturfühler 45 im Träger 41 kann die lokale Temperatur ϑ überwacht werden und zur entsprechenden Auswertung, allenfalls Temperaturregelung, einer Auswertungsanordnung zugeführt werden.

In einer bevorzugten Elektrodenanordnung werden die Merkmale der Elektrodenanordnungen gemäss den Fig. 1 und 2, 3 und 4 kombiniert.

## Patentansprüche

1. Elektrodenanordnung für die Uebertragung oder Ableitung von elektrischen Signalen auf oder von einem Körper eines Lebewesens, welche umfasst:
- einen flächigen forminstabilen, elektrisch isolierenden Träger (31),
- mindestens einen flächigen forminstabilen, elektrisch leitenden Kontaktbereich (33) an der Trägeroberfläche,
- einen elektrischen Anschluss (35, 37) für den Kontaktbereich,
- eine thermisch mit einem Oberflächenbereich der Anordnung gekoppelte Heiz- und/oder Kühlanordnung (44, 44a).

2. Elektrodenanordnung nach Anspruch 1, dadurch gekennzeichnet, dass eine Mehrzahl Kontaktbereiche (33) am Träger (31, 41) vorgesehen ist.

3. Elektrodenanordnung nach einem der Ansprüche 1 und 2, dadurch gekennzeichnet, dass der Kontaktbereich gummielastisch, vorzugsweise aus leitendem Kunststoff, gebildet ist.

4. Elektrodenanordnung nach einem der Ansprüche 1 bis 3, dadurch gekennzeichnet, dass an einer Kontaktierungsoberfläche (33ₒ) von Kontaktbereich und/oder Träger ein Einnehmungsmuster (39) vorgesehen ist.

5. Elektrodenanordnung nach Anspruch 4, dadurch gekennzeichnet, dass das Einnehmungsmuster ein Rinnenmuster ist.

6. Elektrodenanordnung nach einem der Ansprüche 1 bis 5, dadurch gekennzeichnet, dass eine Leitungsanordnung (38) für ein Flüssigmedium vom Träger (31) an den Oberflächenbereich (33ₒ) des Kontaktbereiches und/odere des Trägers ausmündet, zur Drainage und/oder Heizung oder Kühlung und/oder zur Bespülung des Auflagebereiches.

7. Elektrodenanordnung nach einem der Ansprüche 1 bis 6, dadurch gekennzeichnet, dass im Träger eine vorzugsweise flächige Heiz- oder Kühlanordnung (44) eingebettet ist.

8. Elektrodenanordnung nach Anspruch 7, dadurch gekennzeichnet, dass die Heiz- oder Kühlanordnung eine elektrische Heizleiteranordnung (44a) oder ein Leitersystem für ein Flüssigheiz- oder -kühlmedium (44b) umfasst.
